(19) Europäisches Patentamt
European Patent Office
Office européen des brevets

(11) **EP 2 710 139 B2**

(12) **NEW EUROPEAN PATENT SPECIFICATION**
After opposition procedure

(45) Date of publication and mention
of the opposition decision:
**13.04.2022   Bulletin 2022/15**

(45) Mention of the grant of the patent:
**19.09.2018   Bulletin 2018/38**

(21) Application number: **12790268.2**

(22) Date of filing: **18.05.2012**

(51) International Patent Classification (IPC):
*C12Q 1/02* (2006.01)    *G01N 15/14* (2006.01)
*G01N 21/00* (2006.01)

(52) Cooperative Patent Classification (CPC):
**B01L 3/502746; B01L 3/502761; C12N 5/0612;
G01N 15/00; G01N 15/1484; G01N 33/689;
G01N 33/6893;** B01L 2200/0652; B01L 2200/0694;
B01L 2300/0654; B01L 2300/0816;
B01L 2300/0858; B01L 2300/0877;
B01L 2300/0887; G01N 2015/0003;    (Cont.)

(86) International application number:
**PCT/US2012/038680**

(87) International publication number:
**WO 2012/162181 (29.11.2012 Gazette 2012/48)**

(54) **ANALYSIS AND SORTING OF MOTILE CELLS**

ANALYSE UND SORTIERUNG BEWEGLICHER ZELLEN

ANALYSE ET TRI DE CELLULES MOTILES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **20.05.2011   US 201161488300 P**

(43) Date of publication of application:
**26.03.2014   Bulletin 2014/13**

(73) Proprietor: **The Brigham and Women's Hospital,
Inc.
Boston, MA 02115 (US)**

(72) Inventors:
• **DEMIRCI, Utkan
Cambridge
Massachusetts 02139 (US)**
• **ZHANG, Xiaohui
Welstmad
Illinois 60539 (US)**
• **KAYAALP, Emre
New York
New York 10004 (US)**
• **SAFAEE, Hooman
Mississauga
Ontario L5V 2Z1 (CA)**
• **TASOGLU, Savas
Cambridge
Massachusetts 02139 (US)**

(74) Representative: **Forresters IP LLP
Skygarden
Erika-Mann-Straße 11
80636 München (DE)**

(56) References cited:
WO-A2-2010/115167    CA-A1- 2 740 113
US-A1- 2005 026 274    US-A1- 2006 270 021
US-A1- 2008 187 991    US-A1- 2010 291 535

• XIAOHUI ZHANG ET AL: "Lensless imaging for simultaneous microfluidic sperm monitoring and sorting", LAB ON A CHIP, vol. 11, no. 15, 16 June 2011 (2011-06-16) , pages 2535-2540, XP055090272, ISSN: 1473-0197, DOI: 10.1039/c1lc20236g
• X. ZHANG et al.: "Lensless imaging for simultaneous microfluidic sperm monitoring and sorting", Lab on a Chip, vol. 11, no. 11, 2011, pages 2535-2540,
• L. XIE et al.: "Integration of Sperm Motility and Chemotaxis Screening with a Microchannel-Based Device", Clinical Chemistry, vol. 6, no. 8, 2010, pages 1270-1278,
• J. R. JENSEN et al.: "The Effect of a Two-Hour, Room Temperature Incubation of Human Spermatozoa in TEST-Yolk Buffer on the Rate of Fertilization in Vitro", Journal of Assisted Reproduction and Genetics, vol. 21, 2004, pages 169-173,

- **J. M. CUMMINS et al.: "On mammalian sperm dimensions", Journals of Reproduction & Fertility, vol. 75, 1985, pages 153-175,**
- **L. LARSEN et al.: "Computer-assisted semen analysis parameters as predictors for fertility of men from the general population", Human Reproduction, vol. 15, no. 7, 2000, pages 1562-1567,**

(52) Cooperative Patent Classification (CPC): (Cont.)
G01N 2015/0053; G01N 2015/0065;
G01N 2015/149; G01N 2800/367

## Description

### Cross-Reference to Related Application

This application claims the benefit of U.S. Application Serial No. 61/488,300, filed May 20, 2011.

### Statement as to Federally Sponsored Research

This invention was made with Government support under grant numbers R01 AI081534, R21 AI087107, and R21 EB007707 awarded by the National Institutes of Health; an Integration of Medicine and Innovative Technology (CIMIT) grant (DAMD17-02-2-0006) under the U.S. Army Medical Research Acquisition Activity Cooperative Agreement; and a grant (W81XWH-10-1-1050) awarded by the U.S. Army Medical Research & Materiel Command (USAMRMC) and the Telemedicine & Advanced Technology Research Center (TATRC). The Government may have certain rights in the invention.

### Field of the Invention

The invention relates to methods for the analysis and sorting of motile cells, e.g., mammalian sperm cells.

### Background of the Invention

5.3 million American couples of reproductive age (9%) are affected by infertility, among which male factors account for up to 50% of cases. *In vitro* fertilization (IVF), with or without intra-cytoplasmic sperm injection (ICSI) has become the most widely used assisted reproductive technology in modern clinical practice to overcome male infertility challenges. One of the obstacles of IVF and ICSI lies in identifying and isolating the most motile and presumably healthiest sperm from semen samples that may have low sperm counts (oligozoospermia) and/or low sperm motility (oligospermaesthenia).

US2010291535 A1 discusses a method of using a microfluidic chip to sort sperm, wherein the sperm and the medium form a sperm laminar flow and medium laminar flow in the microchannels.

US2008187991 A1 discusses sorting motile particles from non-motile particles in a microfluidic sorting device wherein a stream of sort fluid containing motile and non-motile particles is caused to flow adjacent a media stream in non-turbulent fashion through a sort channel, during which flow motile particles cross the interface between the adjacent flow streams, entering the media stream, and forming a motile particle-depleted sort stream.

US2006270021 A1 discusses an integrated micro fluidic sperm isolation and oocyte insemination device, wherein sperm sorting is performed in a common sort channel wherein more mobile sperm swim across the interface between co-laminar flows of semen and media fluid. Zhang et al.. Lab on a Chip, The Royal Society of Chemistry (2011), volume 11, number 11, pages 2535 to 2540, discloses lensless imaging for simultaneous microfluidic sperm monitoring and sorting. Xie et al., Clinical Chemistry (2010), 56:8, pages 1270 to 1278, discloses integration of sperm motility and chemotaxis screening with a microchannel-based device. US2005/0026274 discloses an apparatus for selecting motile biological species.

### Summary of the Invention

A motile cell sorting and analysis system as described herein can image, track, and sort a population of motile cells, such as sperm, *in situ* and in real time within a space constrained micro fluidic channel. The motile cell sorting and analysis system is a chemical-free and flow-free system capable of rapid, high-throughput cell analysis and sorting. Characteristics of the motile cells, such as the quantity of cells, the average motility, and the motility of specific cells, can be determined. Analysis of such characteristics is important in the diagnosis of various conditions, such as low sperm count (oligozoospermia) and low sperm motility (oligospermasthenia), which may affect fertility. In addition, the most motile cells are passively sorted by the sorting and analysis system without the need for pumps or other peripheral equipment. Samples composed primarily of highly motile sperm are desirable, for instance, for use in assisted reproductive technologies.

The invention is defined in the claims. In a general aspect, a method for sorting motile cells includes introducing an initial population of motile cells into an inlet port of a micro fluidic channel, the initial population of motile cells having a first average motility; incubating the population of motile cells in the microfluidic channel in the absence of flowing media; and collecting a sorted population of motile cells at an outlet port of the microfluidic channel. The sorted population of motile cells has a second average motility higher than the first average motility. The height of the microfluidic channel is 3 to less than 20 times the dimension of the motile cells, and the length of the microfluidic channel is 12 to 15mm.

Embodiments may include one or more of the following.

The motile cells comprise sperm cells, e.g., animal, e.g., mammalian sperm cells.

The method further includes orienting the microfluidic channel horizontally or vertically.

Incubating the population of motile cells includes incubating in the absence of flowing media.

Incubating the population of motile cells includes heating the microfluidic channel to about 37 °C.

Incubating the population of motile cells includes incubating the population of motile cells for a time sufficient to allow a portion of the initial population of motile cells to move along the microfluidic channel, e.g., for about 20-60 minutes, or about 30 minutes.

The height of the microfluidic channel is e.g., about 3 to 10 times the dimension of the motile cells.

The method further includes determining the second average motility, including obtaining a plurality of images, e.g., shadow images, of a collectable population of motile cells in the vicinity of the outlet port, the collectable population of motile cells including the sorted population of motile cells; and analyzing the plurality of images.

The method further includes determining the first average motility based on at least one of an average path velocity (VAP), a straight line velocity (VSL), or a linearity of the initial population of motile cells.

The method further includes determining the second average motility based on at least one of an average path velocity (VAP), a straight line velocity (VSL), or a linearity of the sorted population of motile cells.

Introducing the initial population of motile cells includes suspending the initial population of sperm in a medium at a concentration of at least about $10^3$ sperm/$\mu$L, e.g., at least about $10^4$ sperm/$\mu$L. A concentration of the sorted population of motile cells in a medium is less than or equal to about $1.6 \times 10^3$ sperm/$\mu$L.

In another general aspect, a method for analyzing a population of motile cells includes introducing an initial population of motile cells into an inlet port of a microfluidic channel; incubating the population of motile cells in the microfluidic channel; acquiring a plurality of images of at least a portion of the population of motile cells within the micro fluidic channel; and determining a characteristic of at least a portion of the population of motile cells based on the plurality of images. The height of the microfluidic channel is 3 to less than 20 times the dimension of the motile cells, and the length of the microfluidic channel is 12 to 15mm.

Embodiments may include one or more of the following.

The motile cells include sperm cells, e.g., animal, e.g., mammalian sperm cells.

Acquiring a plurality of images includes acquiring a plurality of shadow images of the at least a portion of the population of motile cells within the microfluidic channel.

The determined characteristic includes at least one of a motility, an average path velocity (VAP), a straight line velocity (VSL), or a linearity.

The determined characteristic includes at least one of (1) a characteristic of a sorted population of motile cells located in the vicinity of an outlet port of the microfluidic channel, and (2) a distribution of the population of motile cells along the length of the microfluidic channel.

Determining a characteristic includes comparing a characteristic of a sorted population of motile cells located in the vicinity of an outlet port of the microfluidic channel with either or both of (1) a characteristic of the initial population of motile cells, and (2) a characteristic of a remaining population of motile cells located in the vicinity of the inlet port after the incubating.

The method further includes determining a sorting capability of the microfluidic channel based on the results of the comparing.

Determining a characteristic includes comparing a characteristic of a remaining population of motile cells located in the vicinity of the inlet port after the incubating with a characteristic of a sorted population of motile cells located in the vicinity of an outlet port of the microfluidic channel after the incubating.

The method further includes determining a health of the initial population of motile cells based on the determined characteristic.

The method further includes collecting a sorted population of motile cells at an outlet port of the micro fluidic channel.

Incubating the population of motile cells includes incubating in the absence of flowing media for a time sufficient to allow a portion of the initial population of sperm to move along the micro fluidic channel, e.g., for about 20-60 minutes, or about 30 minutes.

Incubating the population of motile cells includes incubating the population of sperm for a time sufficient to allow a portion of the initial population of sperm to swim along the microfluidic channel.

The height of the microfluidic channel is e.g., about 3 to 10 times the dimension of the motile cells.

In another general aspect of the disclosure, a device for sorting motile cells includes a microchannel. The height of the microfluidic channel is selected to be three to ten times a dimension of a head of the motile cells. The device further includes an inlet port connected to a first end of the microfluidic channel and configured to receive an initial population of motile cells having a first average motility and an outlet port connected to a second end of the microfluidic channel. The microfluidic channel is configured to provide a sorted population of motile cells at the second end without requiring a fluid flow in the microfluidic channel. The sorted population of motile cells has a second average motility higher than the first average motility.

Embodiments of the disclosure may include one or more of the following.

The motile cells comprise sperm cells, e.g., animal, e.g., mammalian sperm cells. The dimension of the motile cells is a diameter of the head of the sperm cells.

The dimension of the motile cells is a diameter of the motile cells.

The height of the microfluidic channel is selected to be about three to ten times the dimension of the motile cells, e.g., less than about 200 μm, e.g., less than about 60 μm, e.g., about 3-20 μm.

The length of the micro fluidic channel is selected at least in part based on at least one of an incubation time of the motile cells in the channel and a speed of the motile cells, the length is 12-15 mm.

The length of the microchannel is selected at least in part based on at least one of an incubation time of the motile cells in the channel and a swimming speed of the motile cells.

The microchannel is configured to provide the sorted population of motile cells after an incubation time.

The micro fluidic channel has a rectangular cross section, a trapezoidal cross section, a triangular cross section, a circular or oval cross section, a cross section that varies along the length of the microchannel, or a cross section having ridges.

The micro fluidic channel is linear or curved.

The device further includes an imaging system configured to capture a plurality of images of at least a portion of the micro fluidic channel. The imaging system includes a light source configured to illuminate the at least a portion of the microfluidic channel; and a detector configured to detect an image, e.g., a shadow image, of the motile cells in the illuminated portion of the micro fluidic channel.

The device further includes an analysis module configured to determine a characteristic of the motile cells in the imaged portion of the micro fluidic channel based on the captured images.

A "motile cell" is a cell that is able to move spontaneously and actively, e.g., by movement of flagella and/or cilia. Exemplary motile cells for the purpose of the present application include sperm cells, e.g., mammalian sperm cells, neutrophils, macrophages, white blood cells, and certain bacteria.

The systems and methods described herein have a number of advantages. For instance, the motile cell sorting and analysis system facilitates the identification and selection of cells, such as sperm cells, having high motility. A high yield of motile cells is produced without deleterious effects on the cells, even for starting samples having low cell count or low cell motility. The system is simple, compact, inexpensive, and does not require the use of complex instrumentation or peripheral equipment such as tubes or pumps. The results are not operator dependent. The motile cell sorting and analysis system may be useful for fertility clinics wishing to select high motility sperm for use in assisted reproductive technologies and for individuals wishing to check their fertility at home.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

## Brief Description of the Drawings

Fig. 1 is a schematic diagram of an exemplary motile cell sorting and analysis system.

Fig. 2A is a schematic diagram of an exemplary microfluidic chip of a motile cell sorting and analysis system described herein.

Fig. 2B is an exploded view of the exemplary microfluidic chip of Fig. 2A.

Fig. 3 is a schematic diagram of the geometry of an exemplary microchannel.

Fig. 4 is a plot of the average path velocity (VAP) and straight line velocity (VSL) of sorted and non-sorted murine sperm.

Figs. 5A and 5B are bulls-eye plots of murine sperm motility vectors in a horizontally and vertically oriented microchannel, respectively.

Figs. 6A-6C are plots of murine sperm speed, sperm linearity, and sperm acceleration, respectively, in horizontally and vertically oriented microchannels.

Fig. 7 is a plot of experimental and simulated murine sperm distributions within the microchannels of an exemplary microfluidic chip after incubation for 1 hour.

Fig. 8 is a plot of experimental and simulated murine sperm distributions within the microchannels of an exemplary microfluidic chip as a function of incubation time.

Figs. 9A-D are plots of VAP, VSL, and linearity, and percentage of motile murine sperm, respectively, as a function of channel length and incubation time.

Fig. 10 is a plot of murine sperm VAP and VSL and the collectable sperm percentage for sperm sorted as a function of channel length.

Figs. 11A-11D are plots of murine sperm VAP, VSL, linearity, and percentage of motile sperm, respectively, for sperm sorted with a microfluidic chip, sperm sorted by the swim-up technique, and non-sorted sperm.

Fig. 12 is an image showing sperm tracks.

Fig. 13 is a plot of the average mean-squared displacements for the sperm tracks of Fig. 12 fitted to a persistent random walk (PRW) model.

Fig. 14 is a schematic diagram of the trajectory of a sperm performing a persistent random walk (PRW).

Fig. 15 is a plot of the distribution of sperm as a function of channel length.

## Detailed Description

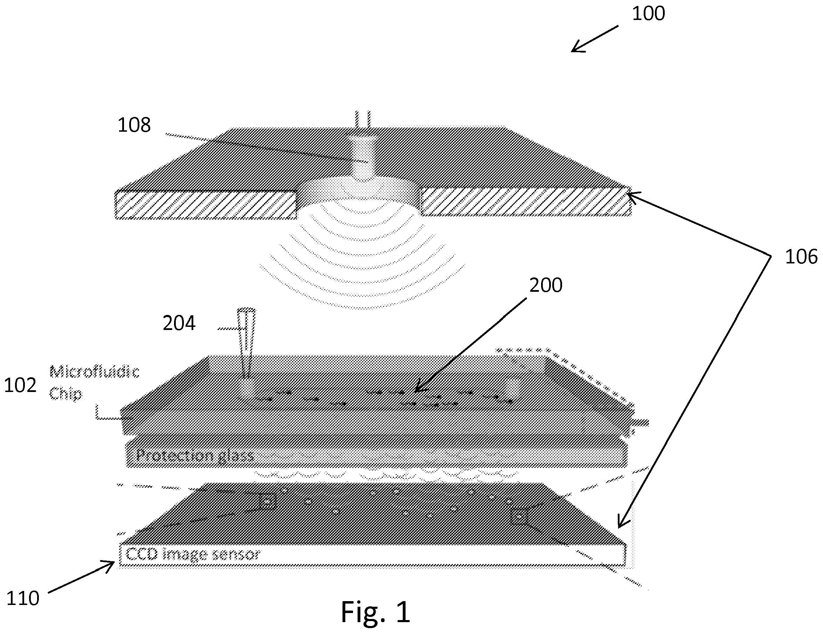

Referring to Fig. 1, a motile cell sorting and analysis system 100 images, tracks, and/or sorts a population of motile cells, such as sperm, *in situ* and in real time within a space constrained micro fluidic channel. The motile cell sorting and analysis system 100 is a chemical-free and flow-free system capable of rapid, high-throughput cell analysis and sorting. Characteristics of the motile cells, such as the quantity of cells, the average motility, and the motility of specific cells, can be determined. Analysis of such characteristics is important in the diagnosis of various conditions, such as low sperm count (oligozoospermia) and low sperm motility (oligospermasthenia), which may affect fertility. In addition, the most motile cells are passively sorted by the sorting and analysis system without the need for pumps or other peripheral equipment. Samples composed primarily of highly motile sperm are desirable, for instance, for use in assisted reproductive technologies.

The exemplary sorting and analysis system 100 includes a microfluidic chip 102, which includes one or more micro fluidic channels 200. In some embodiments, the microfluidic chip 102 is integrated with an imaging system 106, which captures images of sperm within one or more of the microfluidic channels of the microfluidic chip. Analysis of the images allows characteristics of the sperm in the microfluidic channels, such as the number, motility, velocity, acceleration, and/or directionality, to be determined. Furthermore, sperm in a microfluidic channel are sorted as they move (e.g., by swimming or other types of self-propelled motion) along the channel such that a sorted sample of high quality motile sperm can be extracted at the outlet of the channel.

In the following description, the motile cell sorting and analysis system is described with reference to sperm. However, it is to be understood that other motile cells may also be used with the system, such as neutrophils, macrophages, white blood cells, and certain bacteria, such as the bacterium E. coli.

## Structure and fabrication of the microfluidic chip

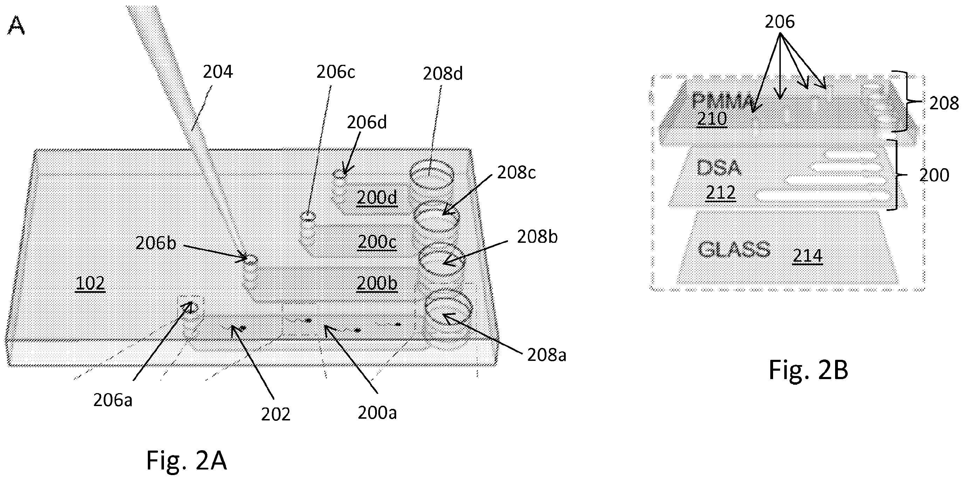

Referring to Figs. 2A and 2B, the microfluidic chip 102 has one or more microfluidic channels 200a, 200b, 200c, 200d. Sperm 202 are introduced, e.g., by injection with a pipette 204, into an inlet port 206a, 206b, 206c, 206d for sorting and/or analysis. After a sufficient incubation period, as discussed below, a sample of sorted sperm is extracted from an outlet port 208a, 208b, 208c, 208d.

The micro fluidic chip 102 is a multilayer structure formed of a base layer 210, an intermediate layer 212, and a cover layer 214. The channels 200 are formed in the intermediate layer 212; the inlet ports 206 and outlet ports 208 are formed in the base layer 210. A first end of each channel 200 is aligned with its corresponding inlet port 206 and a second end of each channel 200 is aligned with its corresponding outlet port 208, thus creating a flow channel from an inlet port 206 to the corresponding outlet port 208 via the channel 200. In some embodiments, the channels 200 extend slightly beyond their respective inlet and outlet ports 206, 208. The channels are sized to accept, e.g., microliter or milliliter volumes of solution containing sperm to be analyzed and/or sorted. The channels may also be further sized and shaped to effect efficient sorting, as discussed below.

The micro fluidic chip is operable for sorting and analysis in either a horizontal configuration (i.e., the channels are oriented horizontally) or a vertical configuration (i.e., the channels are oriented vertically).

The base layer 210 provides structural support to the micro fluidic chip 102 and is formed of a sufficiently rigid material, such as poly(methylmethacrylate) (PMMA; McMaster Carr, Atlanta, GA) in a suitable thickness, such as about 1.5 mm (e.g., about 1 mm to 4 mm). A laser cutter (VersaLaser™, Scottsdale, AZ) is used as needed to cut a larger piece of PMMA into a desired size for the micro fluidic chip (e.g., 24 mm x 40 mm) and to cut holes for the inlet ports 206 and outlet ports 208. In some examples, the outlet ports 208 are larger than the inlet ports 206 to facilitate collection of the sperm that arrive at the outlet end of the channel 200. For instance, in some examples, the inlet ports 206 have a diameter of about 0.375 mm or about 0.65 mm (e.g., about 0.3 mm to 1.2 mm) and the outlet ports have a diameter of about 0.375 mm or about 2 mm (e.g., about 0.3 mm to 3.4 mm.

The intermediate layer 212 is formed of a material that adheres to the base layer 210, such as a double-sided adhesive (DSA) film (iTapestore, Scotch Plains, NJ). Channels 200 are formed by laser cutting polygons, such as rectangular sections, in the intermediate layer 212, which is itself laser cut to the desired size (e.g., the size of the base layer 210). The height of the channels 200 is determined by the thickness of the intermediate layer 212, which is discussed in greater detail below. The length and width of the channels 200 are determined by the length and width, respectively, of the polygons cut into the intermediate layer 212. For instance and as discussed in greater detail below, the channels may be about 1-10 mm wide (e.g., about 4 mm wide) and about 12-15 mm long (e.g., about 15 mm long). In some cases, multiple channels of various lengths and/or widths are formed in the intermediate layer.

After the channels 200 are cut into the intermediate layer 212, the intermediate layer is adhered to the base layer 210 such that the first and second ends of each channel 200 align with or extend slightly beyond the corresponding inlet and outlet ports 206, 210. The cover layer 214, which is, e.g., a glass slide of the same lateral dimensions as the base layer 210 and the intermediate layer 212, is adhered onto the exposed side of the intermediate layer, thereby enclosing the channels 200. In the embodiment depicted in Fig. 2, the microfluidic chip 102 is oriented such that the cover layer 214 is on the bottom. In other embodiments, the microfluidic chip 102 may be oriented such that the cover layer 214 is on the top or such that the top of the channels 200 are open.

In general, the microfluidic chip 102 described herein is passive, i.e., not coupled to an active flow system. That is, motile cells move (e.g., swim) along a microchannel 200 in the microfluidic chip 102 on their own and without being pushed along or otherwise moved by an externally driven fluid flow (e.g., flow of the medium in which the motile cells are suspended).

## Operation of the imaging system

Referring again to Fig. 1, in some embodiments, the sperm sorting and analysis system 100 includes the microfluidic chip 102, the structure of which is described above, integrated with an optional imaging system 106. The integration of the microfluidic chip 102 with the imaging system 106 enables a population of sperm or an individual sperm in one or more of the microfluidic channels 200 to be tracked and analyzed. In some embodiments, the imaging system 106 is a lensless imaging system that achieves automatic and wide field-of-view imaging of one or more channels 200 of the microfluidic chip 102. In other embodiments, the imaging system 104 is a light microscope with, e.g., a 10x objective lens.

The imaging system 106 includes a light source 108, such as a light-emitting diode (LED) or other light source. The light source 108 illuminates one or more channels 200 of the microfluidic chip 102. An image sensor 110 is placed on the opposite side of the microfluidic chip 102 from the light source 108. When light is incident on a channel 200, sperm in the illuminated channel diffract and transmit light. Shadows generated by diffraction of the light by the sperm are imaged by the image sensor 110, generating shadow images of the population of sperm in the channel 200 (i.e., images in which each sperm in the channel 200 is imaged as a shadow). The image sensor may be any appropriate sensor, such as a charge-coupled device (CCD) sensor (Imperx, Boca Raton, FL) or a complementary metal-oxide-semiconductor (CMOS) chip based sensor.

The lensless imaging system 106 generates shadow images of sperm in the channels quickly (e.g., in about one second) and with a wide field of view (FOV). For instance, the FOV of the imaging system 106 may be a few millimeters by a few millimeters (e.g., 4 mm x 5.3 mm) up to as large as a few centimeters by a few centimeters (e.g., 3.725 cm x 2.570 cm), or another size appropriate to image a portion of or the entirety of one or more channels 200 (e.g., up to ten parallel channels).

In some cases, hundreds of thousands of individual sperm may be encompassed by the FOV of the imaging system 106. Furthermore, because of the wide FOV of the imaging system 106, each individual sperm stays within the FOV of the imaging system for a relatively long period of time. Thus, sperm motion and activity can be tracked and analyzed for a large number of sperm, collectively or individually, over a long period of time, enabling accurate statistics to be acquired. In some embodiments, the imaging system 106 is designed to image sperm within the FOV with sufficient contrast and signal-to-noise ratio to be detected or counted individually, which may in some cases result in a sacrifice in spatial resolution.

The images are processed manually and/or automatically using image analysis software (e.g., ImagePro software, Media Cybernetics, Inc., MD) to count, identify, track, and analyze the activity of individual sperm or populations of sperm (e.g., motile sperm) in the imaged channel(s). For instance, to analyze images acquired for sperm distribution in a particular channel, automated counting and identification of the sperm in each image is performed. The count results are compared to diffraction theory, which includes the distance between the active region of the image sensor 110 (e.g., the active surface of a CCD sensor) and the location of the imaged microscopic object (e.g., the sperm cell) as critical parameters. To quantitatively investigate the effect of cell shadow diameter on the detected signal strength, the captured diffraction signatures of the sperm cells are fitted to a model. In the example of a lensless imaging system, the operation of the system can be modeled by numerically solving the Rayleigh-Sommerfeld diffraction equation.

The use of a large area CCD and the incorporation of appropriate software processing, such as video based particle tracking codes, enables a high degree of scalability, such that, for instance, millions of sperm may be monitored and analyzed simultaneously.

## Use of the sperm sorting and analysis system

Sperm suspended in a biocompatible medium, such as Human Tubal Fluid (HTF) or phosphate-buffered saline (PBS), are introduced into a micro fluidic channel 200 of the microfluidic chip 102 via the inlet port 206 of the channel using, e.g., a pipette. The channel may already contain a biocompatible medium. The micro fluidic chip 102 is incubated at 37 °C for a period of time sufficient to allow motile sperm to move along the channel toward the outlet port 208 of the channel. For instance, the incubation period may be about 20-40 minutes, e.g., about 30 minutes, or less than about 1 or 2 hours, or less than an amount of time that would result in sperm exhaustion at the outlet port. After the incubation period, sperm are extracted from the outlet 208, e.g., by using a stripper or pipette, e.g., with a fine tip or by pumping medium into the chip inlet. Because only motile sperm can move along the length of the channel, the sperm extracted from the outlet are motile sperm; the incubation period can be optimized to obtain only high-motility sperm (e.g., by selecting those sperm that arrive at the outlet within a given amount of time). The sperm that remain in the vicinity of the inlet are less motile sperm that were not capable of moving along the entire length of the channel or non-motile sperm that moved only via random motion. Thus, the microfluidic chip 102 achieves simple, passive, flow-free sorting of sperm and enables the extraction of a sample of high-motility sperm.

In addition to sorting, the sperm sorting and analysis system 100 enables various types of analysis to be performed, such as analysis of average sperm motility and tracking and analysis of the paths and motility of individual sperm. For instance, the velocity, acceleration, directionality, or motility of a complete sperm sample or the extracted sorted sperm sample can be quantified, e.g., to identify a high quality sperm sample or to diagnose a problem with the sperm sample (e.g., to diagnose the sample as an oligozoospermic or oligospermaethenic sample).

The sperm sorting system 100 is operable in either a horizontal configuration (i.e., the flow through channels 206 is horizontal) or in a vertical configuration (i.e., the flow through channels 206 is vertical and gravity is used as an additional discriminator in the sorting of sperm). To fertilize an egg *in vivo,* sperm may be required to move towards the egg against gravity due to the anatomy and/or position of the female reproductive system. Thus, conducting sperm analysis and/or sorting in a vertical orientation may offer the ability to more realistically characterize or select sperm than conducting the analysis and/or sorting in a horizontal orientation.

In some embodiments, to reduce error when using a lensless CCD imaging system for sperm counting, the maximum sperm concentration that is resolvable by the imaging system may be estimated based on a model (e.g., as described in Ozcan and Demirci, Lab Chip, 2008, 8, 98-106). For instance, for a CCD area of 4 mm x 5.3 mm, the model predicts a maximum resolvable sperm concentration of $1.6 \times 10^3$ sperm/$\mu$L. When a sperm sample is placed in a microfluidic channel for sorting, especially a long channel, the sperm monitoring may be performed towards outlet end of the channel, where the motile sperm are located. In this region, the concentration of sperm is lower than the concentration of sperm in the vicinity of the inlet. Thus, sperm concentrations higher than the maximum resolvable sperm concentration, such as sperm concentrations that are as high as clinically observed concentrations, may be introduced into the inlet of a channel without reducing the resolving power of the imaging system near the outlet of the channel. As illustrated in the examples below, the ability to introduce sperm concentrations higher than the maximum resolvable sperm concentration was experimentally validated: overlapping shadows for sorted sperm near the outlet of the channel were not observed despite introducing sperm at a concentration of $2 \times 10^4$ sperm/$\mu$L at the inlet.

## Parameters affecting sorting capabilities

Referring again to Fig. 2, when sperm are introduced into microfluidic channel 200 via its inlet port 206, motile sperm move within the microfluidic channel. The microfluidic channel 200 presents a space-confined environment for the sperm, which directs the motile sperm to move along the length of the microfluidic channel toward the outlet port 208. As a result, after a sufficient incubation period, a population of highly motile sperm reaches the vicinity of the outlet port while a population of less motile or non-motile sperm remain at or near their original position in the vicinity of the inlet port 206. The space confinement of sperm within the microfluidic channel thus results in the passive sorting by motility of sperm within the channel.

The geometry of the microfluidic channels may affect the efficiency of sperm sorting within the channel. For instance, the dimensions and shape of the microfluidic channel affect the fluid resistance within the channel. In addition, sperm motion is affected by inter-sperm interactions, which are affected in part by the space available in the channel.

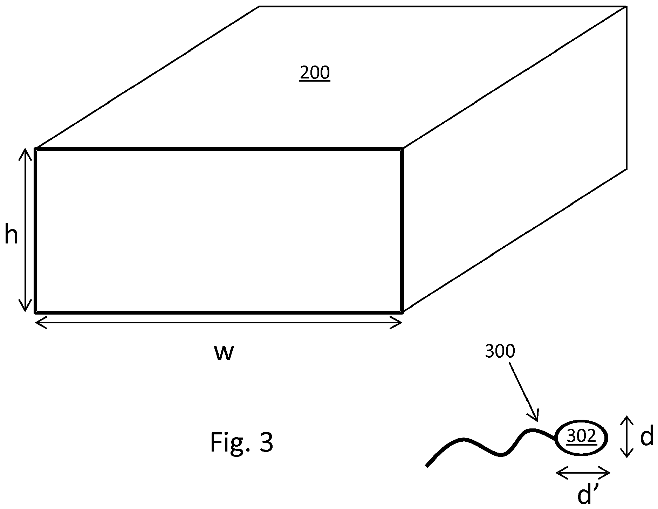

To achieve space confinement of sperm within the microfluidic channel, the height and/or width of the channel (i.e., the thickness of the intermediate layer 212 and the width of the polygon cut into the intermediate layer) may be selected based on the dimensions of the sperm to be sorted within the channel. For instance, referring to Fig. 3, the height h of the channel 200 may be selected to be a small multiple of the dimension d of a head 302 of the type of sperm 300 to be sorted (or more generally, based on a dimension, such as a diameter, of the motile cell to be sorted). In some examples, the height is 3 to less than 20 times the dimension d of the sperm head. In other examples, the width w of the channel 200 may be selected based on the dimension d of the sperm head or a dimension of the motile cell. As shown in Fig. 3, the dimension d is the short diameter of the ovoid sperm head 302. In other embodiments, the dimension d may be the long diameter d' of the ovoid sperm head 302, or the average of the long and short diameters of the sperm head.

More specifically, for human sperm having a head dimension d of about 2-3 $\mu$m, the height h of the channel 200 may be about, e.g., 6-30 $\mu$m, or less than 60 $\mu$m. For rodent (e.g., mouse) sperm having a head dimension d of about 10 $\mu$m, the height h of the channel 200 may be about, e.g., 30-100 $\mu$m, or 50 $\mu$m, or less than 200 $\mu$m. For bovine sperm having a head dimension d of about 4-5 $\mu$m, the height h of the channel 200 may be about, e.g., 10-50 $\mu$m, or less than 100 $\mu$m. For equine sperm having a head dimension d of about 3-4 $\mu$m, the height h of the channel 200 may be about, e.g., 10-40 $\mu$m, or less than 80 $\mu$m. For ram sperm having a head dimension d of about 4 $\mu$m, the height h of the channel 200 may be about, e.g., 10-40 $\mu$m, or less than 80 $\mu$m. For rabbit sperm having a head dimension d of about 4-5 $\mu$m, the height h of the channel 200 may be about, e.g., 10-50 $\mu$m, or less than 100 $\mu$m. For cat sperm having a head dimension d of about 3 $\mu$m, the height h of the channel 200 may be about, e.g., 10-30 $\mu$m, or less than 60 $\mu$m. For dog sperm having a head dimension d of about 3-4 $\mu$m, the height h of the channel 200 may be about, e.g., 10-40 $\mu$m, or less than 80 $\mu$m. For boar sperm having a head dimension d of about 5 $\mu$m, the height h of the channel 200 may be about, e.g., 15-50 $\mu$m, or less than 100 $\mu$m. For sperm from other species, the channel may be sized accordingly. In some embodiments, the dimensions of the channels are determined based on dimensionless quantities determined via simulations of sperm motion within a space constrained environment, as described in more detail below.

In some embodiments, the shape of the microfluidic channel may also be adjusted to effect more efficient sorting of sperm within the channel. For instance, non-straight channels (e.g., curved channels, S-shaped channels, sinusoidal channels, square channels, or angled channels) may be used. The width of the micro fluidic channel may be changed from the inlet port side of the channel to the outlet port side of the channel (e.g., a converging or diverging channel). The sidewalls of the micro fluidic channel may be angled to produce, e.g., a channel having a wide base and a narrow top (e.g., a channel having a trapezoidal cross section), or a channel having another cross section, such as a triangular cross section, a circular or oval cross section, or a cross section of another shape. The cross section may also have ridges, such as ridges formed from a herringbone structure or ridges formed of rectangular fins. The depth of the channel may vary along the length of the channel. Channels of other shapes or having other geometrical features may also be used.

The length of the microfluidic channel 200 is also selected to achieve efficient sorting of sperm within the channel. For a given incubation time, the length of the channel is selected to be short enough such that the motile sperm are able to reach the outlet end of the channel within the incubation time, but long enough such that there is sufficient separation between the motile sperm at the outlet end and the less motile and non-motile sperm at the inlet end of the channel. For instance, for a 30 minute incubation period and for mouse sperm having a velocity of 80-120 $\mu$m/s, a channel length of 12-15 mm may be selected. For the same 30 minute incubation period but for human sperm having a velocity of about 50 $\mu$m/s, a channel length of 12 mm may be selected.

Other design parameters, can also be varied to optimize the sorting capability of the microfluidic channel. For instance, the incubation time of the motile cells in the channel may be varied. Chemical, biological, or temperature gradients may be applied along the channel. Immobilized or dynamic medium and surface parameters, such as, for instance, diffusive transport of nutrients and oxygen, may be varied, e.g., via the presence of other cells such as cumulus cells. Properties of the sorting medium in which the sperm are suspended, such as, for instance, the density, surface tension, porosity, and/or viscosity of the medium, may be varied. Other design parameters may also be varied to affect the sorting capability of the microfluidic channel.

In some cases, some or all of the design parameters for the microfluidic chip 102 are selected according to the specification of a model of sperm motility in a microchannel. The model may simulate the behavior of an individual sperm and/or a population of sperm in a space constrained environment, including, e.g., interactions among sperm and interactions between sperm and the surfaces of the microchannel. The model may incorporate factors such as, e.g., collective hydrodynamic effects, sperm exhaustion, sperm aggregation or other interactions, the cooperativity resulting from hydrodynamic interactions between sperm, the cooperativity resulting from hydrodynamic interactions between a sperm and the channel wall, and/or the wave form of the flagella of the sperm. In addition, the model may incorporate channel geometry parameters, including length, width, height, and shape.

**Examples**

The invention is further described in the following examples. Any examples falling outside the scope of the claims are provided for comparative purposes only.

In general, the following examples demonstrate the ability of the microfluidic chip to sort motile cells, such as sperm. Highly motile cells can be retrieved from the outlet end of the microfluidic channel(s) in the chip while less motile or non-motile cells remain in the channel. The sorting capability of the microfluidic chip depends on a number of parameters, including channel dimensions and incubation time. Characteristics of the motile cells, such as various kinematic parameters of motility, can be determined through analysis of images of the motile cells in the microfluidic channels.

**Example 1 - Sperm sample preparation**

Semen samples were retrieved from B6D2F1 mice aged 7 to 12 weeks from Jackson Laboratories (Bar Harbor, ME). Mice were exposed to $CO_2$ until movement ceased and then euthanized by cervical dislocation. A small incision was made over the midsection, the skin was reflected back, and the peritoneum was entered with sharp dissection to expose the viscera. Both fat pads were pulled down to expose the testes and epididymides. The section of cauda epididymis and vas deferens was excised and placed into a center-well dish containing 300 $\mu$L of Human Tubal Fluid (HTF) (Irvine Scientific, Santa Ana, CA) supplemented with 10 mg mL$^{-1}$ bovine serum albumin (BSA) (Sigma, St Louis, MO). Under a dissection microscope, while holding the epididymis in place with a pair of forceps, incisions were made in the distal parts of the epididymis to allow the sperm to flow out. Spermatozoa were pushed out of the vas deferens by stabilizing the organ with an insulin needle and slowly walking a pair of forceps from one end to the other. The dish was then placed in an incubator (37 °C, 5% $CO_2$) for 10 minutes to allow all sperm to swim out of the epididymis. The epididymis, vas deferens, and larger pieces of debris were manually extracted and discarded.

The sperm suspension was placed in a 0.5 mL Eppendorf tube, and a thin layer of sterile embryo tested mineral oil (Sigma, St Louis, MO) was added on top to prevent evaporation while allowing for gas transfer. The open tube was then placed in an incubator at 37 °C for 30 minutes for capacitation. After capacitation, the tube was gently tapped to mix the sperm suspension.

A 10 $\mu$L sample of capacitated sperm was pipetted out into a new Eppendorf tube and placed in a water bath at 60 °C to obtain dead sperm samples for counting using the Makler® Counting Chamber (Sefi-Medical Instruments, Haifa, Israel). The remaining sperm suspension was adjusted to a concentration of less than 5000 sperm/$\mu$L in HTF-BSA medium and used for the experiments described in the following examples. In particular, the concentration of sperm introduced into the microfluidic chip was in the range of 1500-4000 sperm/$\mu$L, as confirmed by image analysis using ImagePro software (Media Cybernetics, Inc., MD).

In some examples below, the sperm were pre-sorted using the swim-up method prior to introduction into the microfluidic chip. 40 $\mu$L of fresh HTF/BSA medium was added on the top of the sperm suspension in a 0.5 mL Eppendorf tube subsequent to sperm extraction, and a thin layer of sterile embryo tested mineral oil was placed on top of the medium. The tube was placed in an incubator at 37 °C for 1.5 hours to allow for sperm separation. Sperm retrieved from the top of the Eppendorf tube were introduced into the microfluidic chip.

**Example 2 - Sorting of high motility sperm**

To demonstrate the capability of the microfluidic chip to sort sperm based on motility, sperm motilities at the input port and output port were compared to sperm motilities of non-sorted sperm based on sequenced images obtained using an optical microscope. This sorting test used a microfluidic chip having a channel length of 7 mm, a channel width of 4 mm, a channel height of 50 $\mu$m, an inlet port diameter of 0.65 mm, and an outlet port diameter of 2 mm. The large outlet port diameter was designed for easy extraction of sperm from the chanel.

The channel was filled with fresh HTF medium containing 10 mg mL$^{-1}$ BSA. The outlet port was filled with 2 $\mu$L of HTF/BSA medium and a thin layer of mineral oil was placed on top to avoid evaporation. 1 $\mu$L of capacitated sperm was removed after capacitation and added to the inlet port. The micro fluidic chip was placed into an incubator at 37 °C for 30 minutes. At the end of the incubation period, 20 sequenced images were taken of a 1.2 mm x 0.9 mm region at both inlet and outlet ports using a microscope (TE 2000; Nikon, Japan) with a 10x objective lens at the rate of one frame per 0.4-1 seconds using Spot software (Diagnostic Instruments, Inc., version 4.6, Sterling Heights, MI). For validation, the microscope analysis at multiple channel locations was compared to a CCD analysis of the channel.

The sperm count and motility of randomly selected sperm at the inlet port and outlet port were compared to each other, to that of pre-sorted control samples, and to non-sorted sperm based on the sequenced microscope images. The kinematic parameters that define sperm motility, including average path velocity (VAP), straight line velocity (VSL), and linearity (VSL/VAP), were quantified. The VAP is defined as the velocity along the distance that a sperm covers in its average direction of movement during the observation time, while the VSL is defined as the velocity along the straight-line distance between the starting and end points of the sperm's trajectory. Only sperm that showed motility were tracked, although non-motile sperm were also observed.

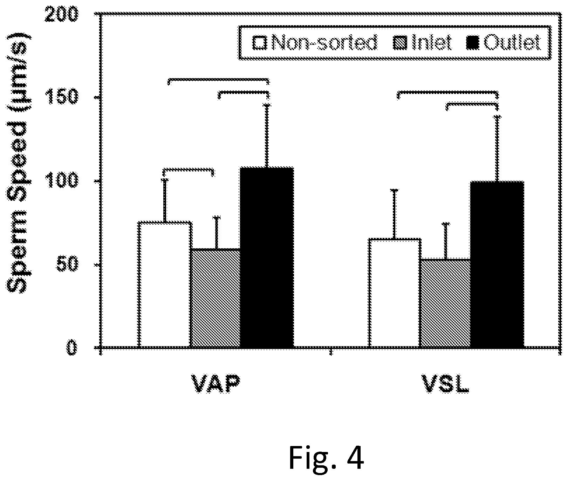

Referring to Fig. 4, the motility (i.e., the VAP and VSL) of sperm at the outlet port was significantly higher than the motility of non-sorted sperm and sperm at the inlet port post-sorting ($p < 0.01$; n = 33-66; brackets indicate statistical significance with $p < 0.01$ between the groups).

The results of the sorting test indicate that the microfluidic chip can successfully sort the most motile sperm, which can be collected at the outlet port after the sorting process is complete, e.g., by a stripper tip or by pumping medium from the inlet port. Furthermore, given the wide range of sperm velocities even after sorting, single chip based processing and monitoring may enable the separation of the highest quality motile sperm utilizing either vertical or horizontal configurations.

**Example 3 - Effect of channel orientation on sperm sorting**

To determine the effect of channel orientation on sperm motility, sperm motion in both horizontal and vertical channel orientations was recorded. The channel had a length of 7 mm, a width of 4 mm, and a height of 50 $\mu$m. The channel was filled with fresh HTF medium supplemented with 10 mg mL$^{-1}$ of BSA. 1 $\mu$L of sperm sample was taken from the very top of a swim-up column as described above and pipetted into the input port of the channel. Fifteen sequenced shadow images were recorded using a lensless CCD sensor at a rate of one frame per second. The CCD sensor covered the entire channel such that all of the sperm in the channel were recorded. Motile sperm were identified and tracked using Photoshop (Adobe, San Jose, CA).

To image sperm in the vertical configuration, the microfluidic chip was clamped to the CCD sensor and the entire system was rotated by 90 degrees. Once the micro fluidic chip was situated vertically, the above preparation and imaging process was repeated using sperm from the same male donor mouse, keeping the system in the vertical orientation for the duration of the imaging.

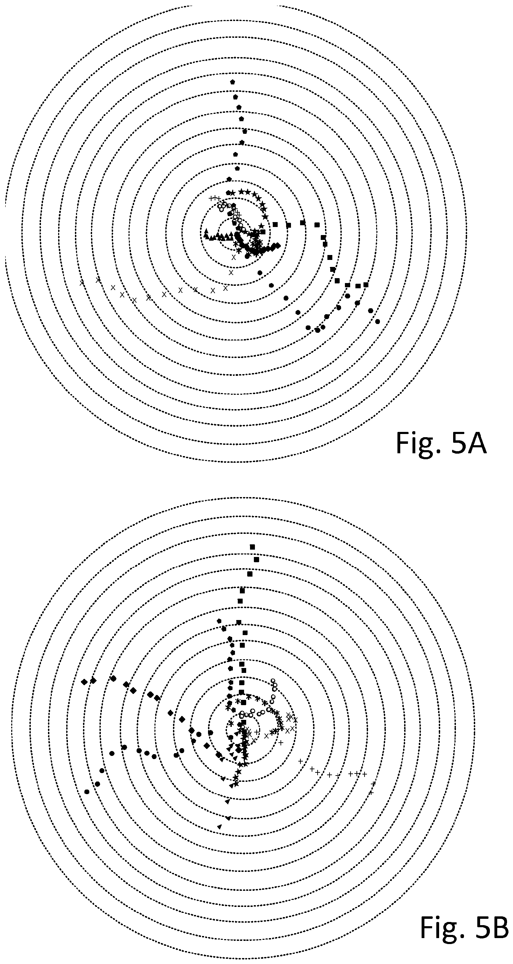

A motility analysis was performed for ten sperm randomly selected from each configuration. In particular, sperm motion in both horizontal and vertical orientations was recorded and the results were displayed as motility vectors in bull's eye plots, as shown in Figs. 5A and 5B, respectively. The distance between adjacent concentric circles is 100 $\mu$m. In both configurations, sperm displayed great diversity in their patterns of motion and direction.

To further characterize sperm motion in both horizontal and vertical orientations, the sperm motion paths were tracked and the travel distance was measured using ImagePro software (Media Cybernetics, Inc., MD). The kinematic parameters that define sperm motility, including average path velocity (VAP), straight line velocity (VSL), and linearity (VSL/VAP), were quantified. Only sperm that showed motility were tracked, although non-motile sperm were also observed.

Referring to Table 1, the VAP, VSL, and linearity were quantified for each of the selected ten horizontal and ten vertical sperm. As can be seen, sperm cells H2 and H9 showed the highest motilities in the horizontal configuration and sperm cells V1 and V2 showed the highest motilities in the vertical configuration.

Table 1. Sperm motility parameters for selected spermatozoa.

| Sperm | Average Path Velocity (VAP) | Straight Line Velocity (VSL) | Linearity (VSL/VAP) |
|---|---|---|---|
| H1 | 69.10 | 57.14 | 0.83 |
| H2 | 85.19 | 66.07 | 0.78 |
| H3 | 19.95 | 13.57 | 0.68 |
| H4 | 21.56 | 17.86 | 0.83 |
| H5 | 36.82 | 17.86 | 0.48 |
| H6 | 67.36 | 61.43 | 0.91 |
| H7 | 21.61 | 14.29 | 0.66 |
| H8 | 26.33 | 18.57 | 0.71 |
| H9 | 80.05 | 65.36 | 0.82 |
| H10 | 29.57 | 8.57 | 0.29 |
| V1 | 90.03 | 75.00 | 0.83 |
| V2 | 86.75 | 67.86 | 0.78 |

(continued)

| Sperm | Average Path Velocity (VAP) | Straight Line Velocity (VSL) | Linearity (VSL/VAP) |
|---|---|---|---|
| V3 | 47.76 | 39.29 | 0.82 |
| V4 | 92 | 66.07 | 0.71 |
| V5 | 28.72 | 22.14 | 0.77 |
| V6 | 55.31 | 45.71 | 0.83 |
| V7 | 37.09 | 23.57 | 0.64 |
| V8 | 69.9 | 57.50 | 0.82 |
| V9 | 41.50 | 19.29 | 0.46 |
| V10 | 53.18 | 10.71 | 0.20 |

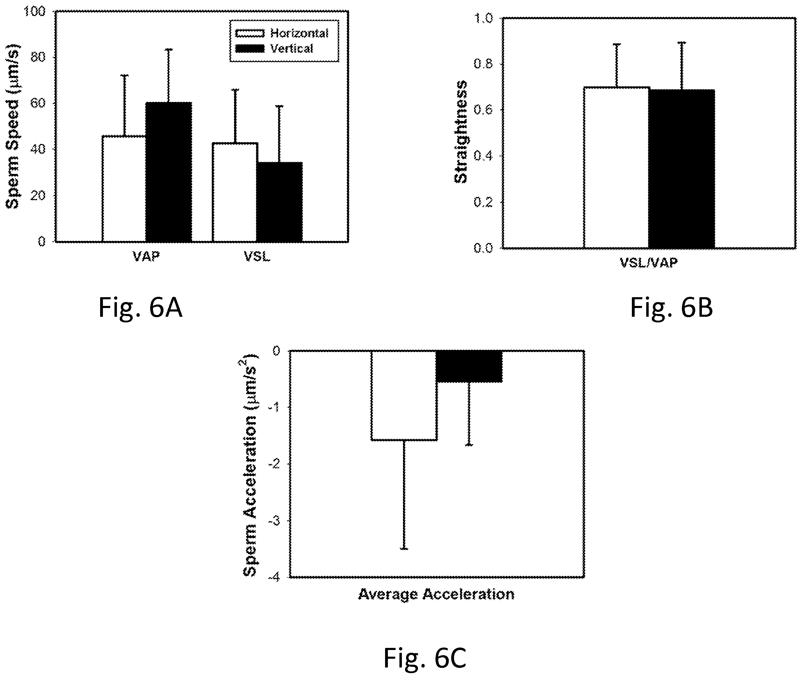

Referring to Figs. 6A-6C, the sperm imaged in both horizontal and vertical configurations were analyzed statistically for VAP, VSL (Fig. 6A), linearity (Fig. 6B), and acceleration (Fig. 6C). For a small set of mobile capacitated sperm that were monitored for a short period of time, imaging in horizontal and vertical configurations did not result in a statistically significant difference ($p > 0.05$), thus demonstrating that the microfluidic chip can be used substantially interchangeably in either configuration. The sperm acceleration, in contrast, spanned a broader range of values in the vertical configuration (-75 to 90 $\mu$ s$^{-2}$) than in the horizontal configuration (-50 to 30 $\mu$ s$^{-2}$).

For this example and further examples described below, VAP, VSL, and linearity were statistically analyzed for significance of the difference between the following groups using a two-sample parametric student t-test with statistical significance set at 0.05 ($p < 0.05$): (i) sperm at the inlets and outlets after sorting; (2) sperm sorted by the microfluidic chip with a 30 minute incubation time and sperm sorted by the swim-up technique; and (3) sperm sorted by the microfluidic chip with a 30 minute incubation time and non-sorted sperm. The statistical significance threshold was set at 0.05 ($p < 0.05$) for all tests and data were presented as average $\pm$ standard error (SEM). To further assess the sorting potential of the microfluidic chip, VAP and VSL were also analyzed for the non-sorted condition (n = 33), inlet (n = 59), and outlet (n = 66) measurements with One-Way Analysis of Variance (ANOVA) with the Tukey's post-hoc multiple comparison test with statistical significance threshold set at 0.01 ($p < 0.01$). The normality of the data collected was analyzed with the Anderson-Darling test.

### Example 4 - Effect of incubation time on sperm sorting

To optimize the incubation time for sperm sorting using the microfluidic chip, sperm distribution throughout a channel 20 mm long, 4 mm wide, and 50 $\mu$m high was imaged and analyzed for various incubation times.

The channel was filled with fresh HTF medium containing 10 mg mL$^{-1}$ BSA. The outlet port was filled with 2 $\mu$L of HTF/BSA medium; a thin layer of mineral oil was placed on top to avoid evaporation. 1 $\mu$L of sperm sample diluted to a density of 1500-4000 sperm/$\mu$L was introduced into the channel from the inlet port, and the inlet port was covered with a thin layer of sterile embryo tested mineral oil to avoid evaporation. The microfluidic chip was placed into an incubator at 37 °C for various incubation times, including 5 minutes, 15 minutes, 30 minutes, and 1 hour.

After incubation, the sperm distribution within the channel was imaged using a microscope (Carl Zeiss Micro-Imaging, LLC, Thornwood, NY) with an automated stage controlled by AxioVision software (Carl Zeiss MicroImaging). Automated and manual analysis was used to analyze the sperm distribution. For regions close to the inlet, where the sperm concentration is relatively high, the sperm were automatically counted using ImagePro software. For regions of lower sperm concentration (e.g., near the outlet), manual counting was used.

A control distribution experiment was also performed by placing heat-killed (20 minutes at 60 °C) sperm and measuring sperm distribution within the channel after incubation for 5 minutes and 1 hour.

To investigate the effects of exhaustion time of sperm and the role of the initial percentage of dead sperm on the observed sperm distribution within the channel, the experimental sperm distribution for each incubation time was compared with the control sperm distributions and with predictions of a coarse-grained model of sperm motility in the channel. The active motility of the sperm was modeled as a persistent random walk (PRW); dead sperm were modeled as moving only by Brownian forces mimicked by an isotropic random walk (as discussed above).

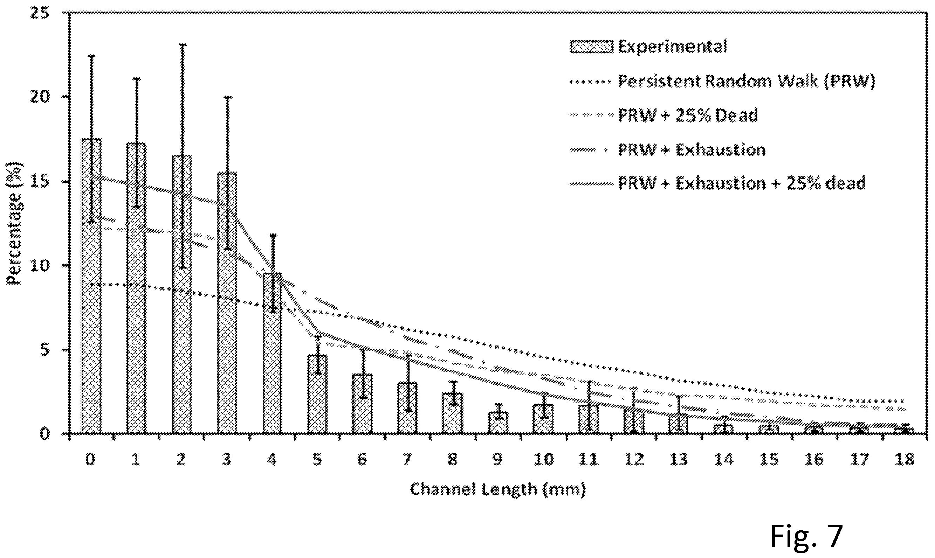

Fig. 7 shows the experimental sperm distribution at various points along the channel after incubation for 1 hour. The experimental results are compared with the PRW model with various parameters: (1) PRW model; (2) PRW with 25% of sperm initially dead; (3) PRW including 30 minutes average incubation time ($\pm$ 15 minutes); and (4) PRW including both 30 minutes incubation time and 25% of sperm initially dead. Error bars refer to average $\pm$ standard error. The

experimental results best match the PRW model in which the sperm had an average exhaustion time (incubation time) of 30 minutes and in which 25% of sperm were initially dead. These results are consistent with experimental measurements indicating that 20% of sperm in a given sperm sample are dead immediately prior to injecting the sample into the inlet port.

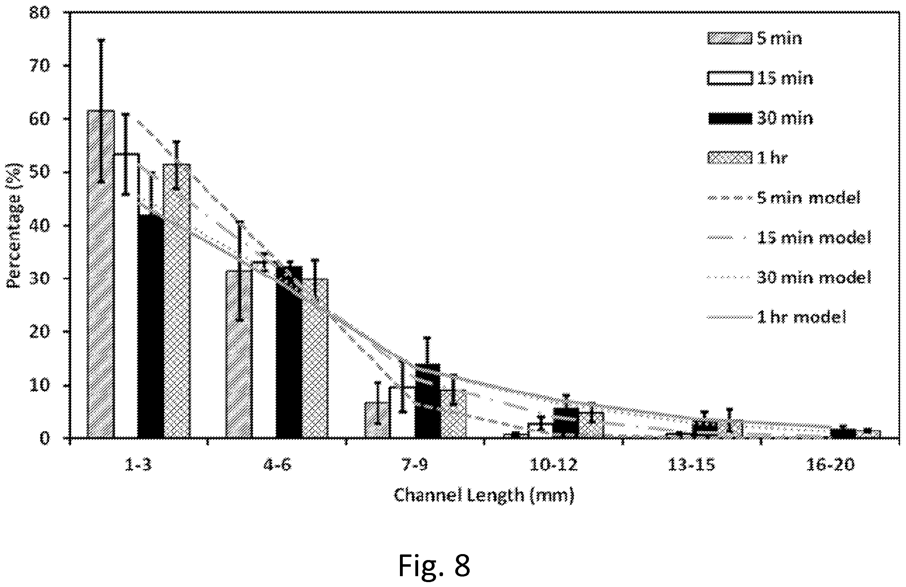

Fig. 8 shows the experimental sperm distribution within the channel after incubation periods of 5 minutes, 15 minutes, 30 minutes, and 1 hour. The experimental distribution for each incubation time was compared with the PRW model including the same incubation time and having 25% of sperm initially dead. Error bars refer to average ± standard error. A shift of sperm distribution from the inlet port towards the outlet port was observed within 30 minutes of incubation, indicating that a portion of the sperm swam away from the inlet port and towards the outlet port during incubation. This distribution shift peaked at the end of the 30 minute incubation period. More particularly, the percentage of sperm in the channel locations 7-20 mm increased up to the 30 minute incubation period, then decreased for longer incubation times. A similar, but reverse, trend was observed for the sperm distribution in the channel locations 1-3 mm. These results can be attributed to the exhaustion of sperm.

The simulation results for a 30 minute incubation period (standard deviation ± 15 minutes) show the best agreement to the experimental results.

## Example 5 - Effect of channel length on sperm sorting

The effect of channel length on sperm sorting capability was determined by comparing characteristics of sperm at the inlet port and outlet port of channels of various lengths.

Sperm samples were prepared and channels filled as described above in Example 4. Channel lengths of 7 mm, 10 mm, 10 mm, and 20 mm were used. For each channel length, incubation times of 30 minutes and 1 hour were applied.

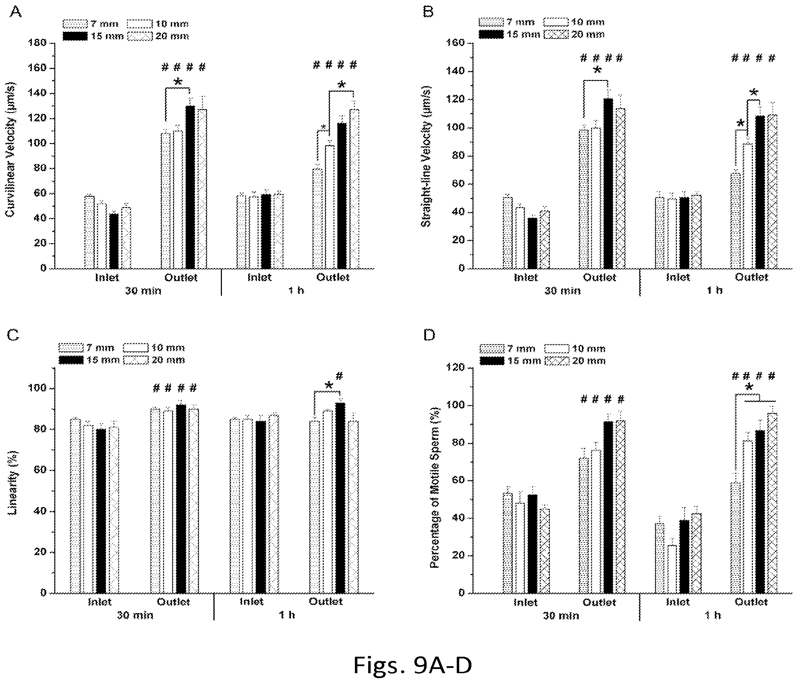

Referring to Fig. 9A-D and Table 2, the VAP, VSL, linearity, and percentage of motile sperm at the inlet and outlet after 30 minutes or 1 hour of incubation time are shown for each channel length. As discussed in greater detail below, all channel lengths investigated demonstrated sorting capability, although the sperm motility (VAP, VSL, and linearity) and percentage of motile sperm varied among the channel lengths. Statistical significance between channel lengths is marked with a * and statistical significance between inlet and outlet is marked with a #. Data are presented as average ± standard error (N = 22-109).

Table 2. Fold change between the inlet and outlet of the SCMS system in average path velocity (VAP), straight-line velocity (VSL), linearity, and percentage motility of the sperm, for different channel lengths and incubation times.

| | Fold change between inlet and outlet in SCMS system | | | | | | | |
|---|---|---|---|---|---|---|---|---|
| **Channel length** | 7 mm | | 10 mm | | 15mm | | 20 mm | |
| **Incubation time** | 30 min | 1 hour | 30 min | 1 hour | 30 min | 1 hour | 30 min | 1 hour |
| **VAP** | 1.9 | 1.4 | 2.1 | 1.7 | 3.0 | 2.0 | 2.6 | 2.1 |
| **VSL** | 1.9 | 1.3 | 2.3 | 1.8 | 3.8 | 2.1 | 2.8 | 2.1 |
| **Linearity** | 1.1 | 1.0 | 1.1 | 1.0 | 1.2 | 1.1 | 1.1 | 1.0 |
| **Percentage of motility** | 1.3 | 1.6 | 1.6 | 3.1 | 1.7 | 2.2 | 2.0 | 2.3 |

Referring specifically to Figs. 9A-9B and Table 2, for a 30 minute incubation period, the VAP and VSL of sperm at the outlets were 1.9, 2.1, 3.0, and 2.6-fold; and 1.9, 2.3, 3.8, and 2.8-fold higher than the VAP and VSL of sperm at the inlets for 7 mm, 10 mm, 15 mm, and 20 mm long channels, respectively. When the incubation period was increased to 1 hour, the VAP and VSL of sperm at the outlets decreased to 1.4, 1.7, 2.0, and 2.1-fold; and 1.3, 1.8, 2.1, and 2.1-fold higher than the VAP and VSL of sperm at the inlets for 7 mm, 10 mm, 15 mm, and 20 mm long channels, respectively.

Referring to Fig. 9C and Table 2, significant differences in linearity of sperm at the inlets and outlets were observed for all channel lengths for the 30 minute incubation period. However, for an incubation period of 1 hour, a significant difference in linearity of sperm at the inlets and outlets was only observed for the 15 mm channel length; no significant difference was observed for the 7 mm, 10 mm, and 20 mm channels. These results demonstrate that when the incubation time is increased beyond an optimal incubation time (in this case, 30 minutes), sperm with less motility and linearity have a higher chance of reaching the outlet of a short channel. The decreased linearity of sperm at the outlet of the 20 mm channel may be attributed to exhaustion.

Referring to Fig. 9D and Table 2, significant differences in the percentage of motile sperm at the inlets and outlets were observed for all channel lengths for both the 30 minute incubation period and the 1 hour incubation period, where the percentage of motile sperm was defined as the fraction of motile sperm relative to the total sperm count. For the 30 minute incubation period, the percentage of motile sperm at the outlets was 1.3, 1.6, 1.7, and 2.0-fold higher than the percentage of motile sperm at the inlets for 7 mm, 10 mm, 15 mm, and 20 mm long channels, respectively. When the incubation period was increased to 1 hour, the percentage of motile sperm at the outlets was 1.6, 3.1, 2.2, and 2.3-fold higher than the percentage of motile sperm at the inlets for 7 mm, 10 mm, 15 mm, and 20 mm long channels, respectively. The increase in the percentage of motile sperm at the outlets for the 1 hour incubation period as compared to the 30 minute incubation period may be due to more of the motile sperm moving away from the inlets during the longer incubation period.

In this example and in other examples related to the effect of channel length, the significance of channel length, geometry, and surface patterns on exhaustion and sperm sorting outcome was tested via non-parametric one-way analysis of variance (ANOVA) with Tukey post-hoc comparisons.

## Example 6 - Effect of channel length on sperm sorting efficiency

The effect of channel length on sperm sorting efficiency was investigated by comparing sperm motility and percentage of motile sperm after sorting using various channel lengths.

Referring again to Fig. 9A and 9B, for a 30 minute incubation period, sperm sorted with a 15 mm long channel showed significant higher VAP (130.0 $\pm$ 31.1 $\mu$m/s) and VSL (120.6 $\pm$ 31.6 $\mu$m/s) than sperm sorted with a 7 mm long channel (VAP: 107.9 $\pm$ 28.1 $\mu$m/s; VSL: 98.3 $\pm$ 30.3 $\mu$m/s) and than sperm sorted with a 10 mm long channel (VAP: 109.8 $\pm$ 26.9 $\mu$m/s; VSL: 100.0 $\pm$ 30.3 $\mu$m/s). However, increasing the channel length to 20 mm did not further improve sperm sorting (VAP: 127.2 $\pm$ 41.3 $\mu$m/s; VSL: 113.7 $\pm$ 38.0 $\mu$m/s) over the sorting by the 15 mm long channel. These data indicated that an increase in channel length up to 15 mm allowed motile sperm to move farther within the channel than low-motility or non-motile sperm, due to differences in sperm velocity, thus resulting in improved sperm sorting.

For the 1 hour incubation period, the 15 mm long channel (VSL: 108.5 $\pm$ 27.8 $\mu$m/s) still demonstrated a better sorting capability than did the 7 mm long channel (VSL: 67.7 $\pm$ 25.2 $\mu$m/s) or the 10 mm long channel (VSL: 88.6 $\pm$ 27.8 $\mu$m/s). However, sperm sorted with the 20 mm long channel displayed significantly higher VAP (VAP: 127.3 $\pm$ 24.1 $\mu$m/s) than sperm sorted with the 7 mm long channel (VAP: 79.6 $\pm$ 23.6 $\mu$m/s) and than sperm sorted with the 10 mm long channel (VAP: 98.4 $\pm$ 27.1 $\mu$m/s).

No significant improvement in sperm velocity was observed between the 30 minute incubation period and the 1 hour incubation period.

Referring to Fig. 9C, for sperm linearity, no significant difference was observed among different channel lengths for the 30 minute incubation period. However, when the incubation time was increased to 1 hour, a significant reduction in the linearity as compared to the 30 minute incubation time was observed for sperm shorted with short channels (7 mm; p < 0.05), but not for sperm sorted with longer channels (10 mm, 15 mm, and 20 mm). This result may be attributed to sperm with lower motility reaching the outlet of a short channel given sufficient incubation time.

Referring to Fig. 9D, a statistical analysis was performed to identify the percentage of motile sperm at the inlet and outlet of each channel. For the 30 minute incubation period, sperm sorted using different channel lengths did not show a statistical difference in the percentage of motile sperm at the inlet and outlet of the channel. When the incubation time was increased to 1 hour, a significant decrease in the percentage of motile sperm at the inlet and outlet was observed for the 7 mm long channel as compared to the longer channels. A decrease in the percentage of motile sperm was also observed for the 7 mm long channel with a 1 hour incubation period as compared to the same channel with a 30 mm incubation period. However, increasing the incubation time to 1 hour did not result in a significant effect on the percentage of motile sperm for longer channels. These results may be attributed to sperm with extremely low motility reaching the outlet of the 7 mm channel given sufficient incubation time. However, the possibility of low motility sperm reaching the outlet of a longer channel (e.g., the 15 mm or 20 mm channel), even with 1 hour of incubation time, was small.

Furthermore, the percentage of sorted sperm that can be collected from the microfluidic chip (referred to as the "collectable sperm percentage") was assessed relative to the total sperm introduced into the channel for each channel length, with a 30 minute incubation period. In particular, the collectable sperm percentage in a channel was calculated based on the sperm distribution within the channel for a 30 minute incubation period. The volumes of sorted sperm that are to be collected from the 7 mm, 10 mm, 15 mm, and 20 mm long channels were 0.2 $\mu$L, 0.6 $\mu$L, 1 $\mu$L, and 1 $\mu$L, respectively (equivalent to the volume of sperm samples in the last 1 mm, 3 mm, 5 mm, and 5 mm of the channels), in addition to the media in the outlet (3 $\mu$L). The collectable sperm percentage was calculated by dividing the total sperm count introduced into the channel by the sorted sperm that would be collected from the channel in the given volume.

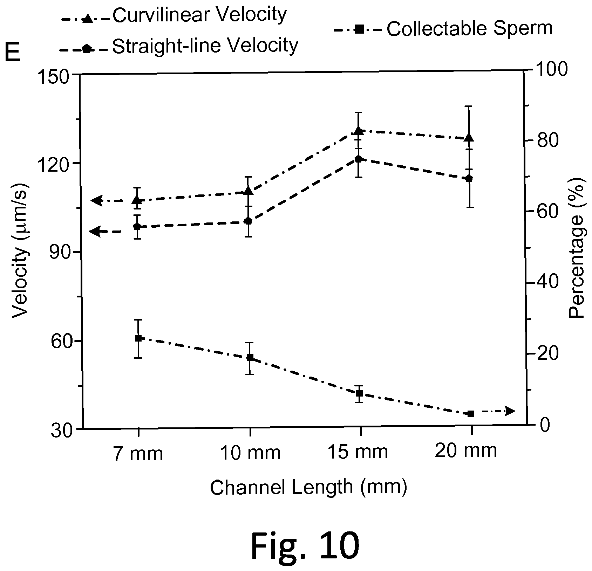

As shown in Fig. 10, the percentages of sperm within a collectable range close the outlet were 25.6%, 19.7%, 9.4%, and 3.3% for the 7 mm, 10 mm, 15 mm, and 20 mm long channels, respectively. These results indicated that the number of sperm within the collectable range close to the outlet decreased as the channel length increased, an effect that may be due to sperm with lower motility being collected from a short channel along with sperm with higher motility.

Data were presented as average $\pm$ standard error with N = 3.

Based on the results above, it can be concluded that the optimal channel length and incubation time are 15 mm and 30 minutes, respectively, to achieve efficient sperm sorting.

**Example 7 - Comparison of microfluidic chip sorting with conventional swim-up sorting**

The characteristics of sperm sorted by a microfluidic chip having a channel 15 mm long, 4 mm wide, and 50 $\mu$m high and a 30 minute incubation period were compared to the characteristics of sperm sorted by a conventional swim-up technique with a 30 minute incubation period and to a sample of non-sorted sperm.

Sperm sorting using the 15 mm long channel was conducted as described above.

Sperm samples were prepared for swim-up sorting by incubating a sperm sample for 30 minutes to allow the sperm to capacitate, followed by pipetting 90 $\mu$L of sperm sample into an Eppendorf tube and diluting the sample to a concentration less than 5000 sperm/$\mu$L. 60 $\mu$L of fresh HTF-BSA medium was added on top of the sperm suspension to create a debris-free overlying medium. A thin layer of sterile mineral oil was added on top of the HTF-BSA medium to prevent evaporation. The Eppendorf tube was placed into an incubator at 37 °C and incubated for 30 minutes or 1 hour. After incubation, 5 $\mu$L of sperm sample was taken from the very top of the medium for motility analysis.

To analyze the sperm sorted using the swim-up technique, sperm samples were placed onto a PMMA slide (24 mm x 60 mm) for imaging, thus eliminating the effect of the substrate on sperm movement measurements between the swim-up technique and the microfluidic chip technique. In particular, 5 $\mu$L of sperm sample was added to a 10 $\mu$L drop of HTF-BSA medium placed on the PMMA substrate. Two strips of DSA film (3 mm x 25 mm) were placed on the PMMA. The sperm-medium drop was covered with a glass slide (25 mm x 25 mm); the DSA film positioned between the glass slide and the PMMA substrate created a space for the sperm to move freely.

The sperm sample on the PMMA was imaged under a microscope and analyzed to determine sperm motility and percentage of motile sperm. 25 sequential microscope images (TE 2000; Nikon, Japan) were acquired using a 10x objective at an average rate of one frame per 0.6 seconds using Spot software (Diagnostic Instruments Inc., version 4.6).

These sample preparation and imaging techniques were also used to prepare the control sample of non-sorted sperm.

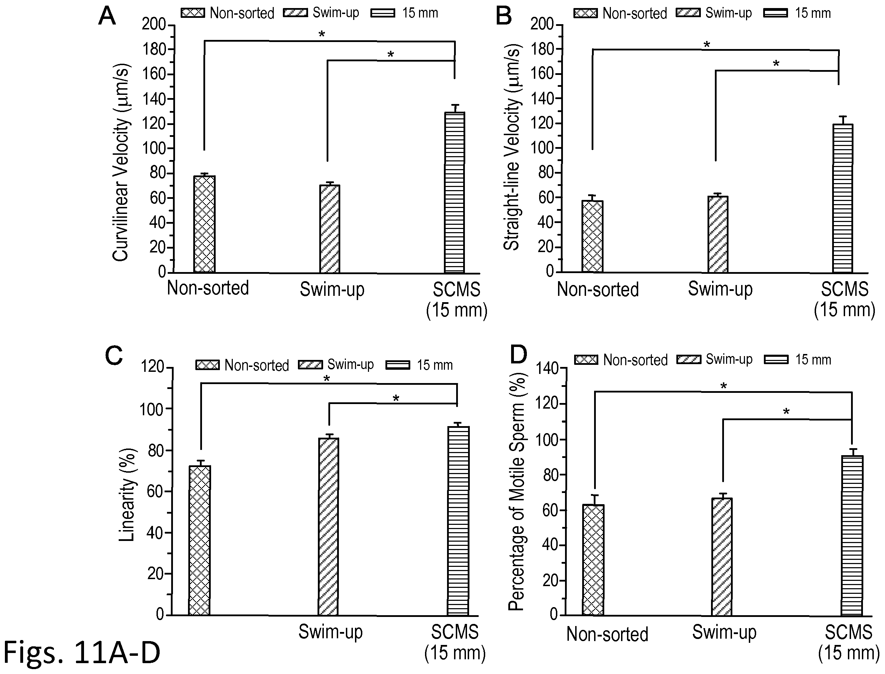

Referring to Fig. 11A-11D, the 15 mm long channel resulted in sorted sperm having a significantly higher motility (VSL, VSL, and linearity) and percentage of motile sperm than sperm sorted by the swim-up technique and than non-sorted sperm, demonstrating that the microfluidic chip is an effective way to sort high motility sperm. Data were presented as average $\pm$ standard error with N = 4-7.

**Example 8 - Theoretical analysis of sperm tracks**

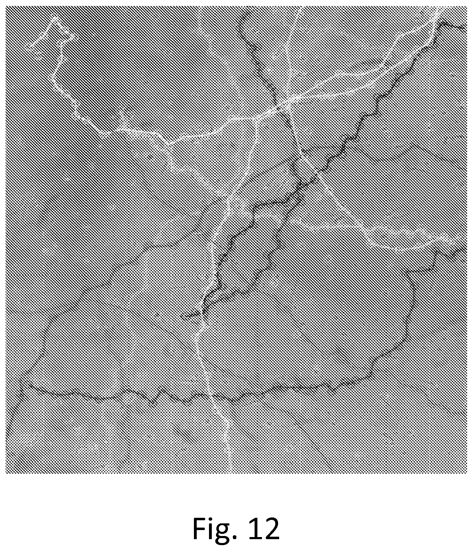

Once the individual sperm were tracked, the mean square displacement (MSD) of each sperm was calculated. Fig. 12 shows sample sperm trajectories obtained using ImageJ software with MTrackJ Plugin (Meijering, Dzyubachyk, Smal. Methods for Cell and Particle Tracking. Methods in Enzymology, vol. 504, ch. 9, February 2012, pp. 183-200).

Considering only the motion of the sperm in the x-y plane, the MSD is given by

$$\langle d^2(t) \rangle = \langle (x(t) - x_0)^2 + (y(t) - y_0)^2 \rangle,$$

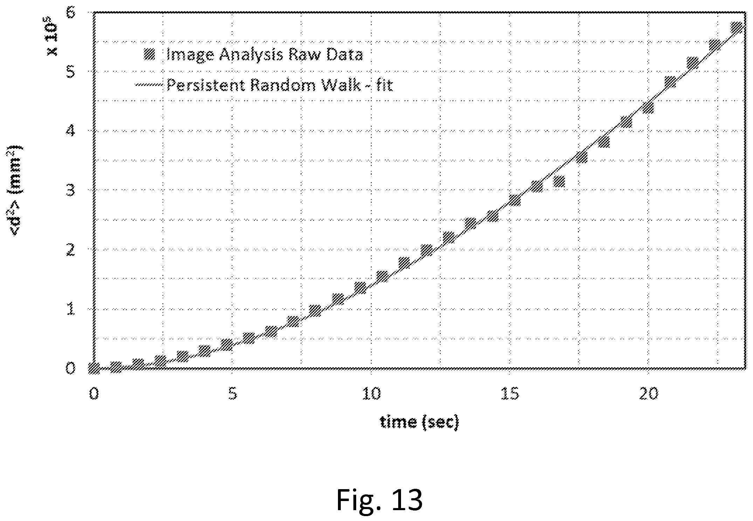

where, x(t) and y(t) correspond to the coordinates, and, $x_0$ and $y_0$ are the origins of each sperm track. The brackets denote averages over different sperm tracks. The resulting MSD as a function of time averaged over 20 data sets is shown in FIG 13. At short times, the motion of an individual sperm is ballistic ($\sim t^2$), and at long times it is diffusive ($\sim t$). Such motility is described by the Persistent Random Walk (PRW) model. In a PRW, the MSD is given by

$$\langle d^2(t) \rangle = 2S^2 P \left[ t - P \left( 1 - e^{-t/P} \right) \right],$$

where S denotes the velocity of the random walker, and P corresponds to the persistence time. In the limit of short times, $t << P,$ the MSD in the PRW model reduces to

$$\langle d^2(t) \rangle \cong S^2 t^2.$$

In the limit of long times, i.e. $t >> P,$ the MSD is given by

$$\langle d^2(t) \rangle \cong 2S^2 Pt.$$

A random motility coefficient, similar to a diffusion coefficient, is given by

$$\mu = \lim_{t \to \infty} \frac{\langle d^2(t) \rangle}{4t} = S^2 P / 2. \quad (5)$$

The MSD data shown in Fig. 13 can be successfully fitted to the above expression for the MSD in a PRW model to give $S \approx 42$ $\mu m/s$ for the velocity and $P \approx 13$ s for the persistence time. The random motility coefficient is then given by $\mu \approx 0.011$ $mm^2/s$.

## Example 9 - Simulations of sperm motility

The motion of active mouse sperm in a microchannel was modeled as a persistent random walk (PRW), as described above. The simulations were restricted to two dimensions, consistent with the 50 $\mu$m thickness of the channel. The channel measures 20 mm by 4 mm, mimicking the experimental setup. The initial distribution of sperm is shown in Fig. 12.

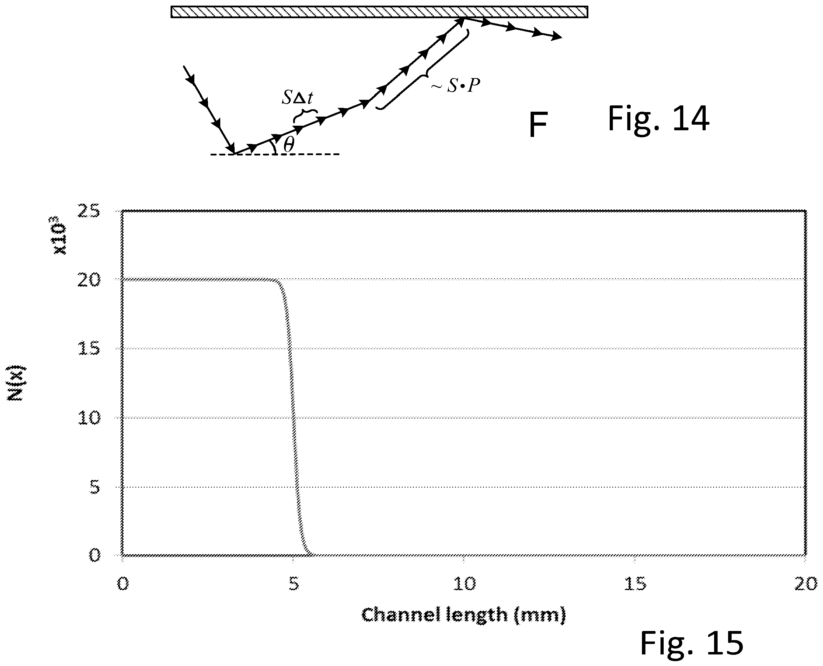

In the model, the active sperm moves in a given random direction $\theta(t)$ (Fig. 14) with velocity, $S(t) = S \cos\theta(t)\hat{i} + S \sin\theta(t)\hat{j}$, for an average duration of P, before switching direction. $\theta(t)$ is chosen from a uniform distribution on the interval $(0, 2\pi]$. If the simulation time step is denoted with $\Delta t$ (chosen as 1 s), then the probability of choosing a new $\theta(t)$ direction for the sperm at every time step is $\Delta t /P$. This means that the sperm persists with constant $\theta(t)$ for an average $P/\Delta t$ time steps before changing orientation.

In the simulations, the S and P values obtained from the fits to experimental tracking data were used (see Example 8). The resulting equations of motion for the position (x,y) of the sperm are

$$x(t + \Delta t) = x(t) + S \cos\theta(t) \, \Delta t$$

$$y(t + \Delta t) = y(t) + S \sin\theta(t) \, \Delta t.$$

When the sperm is not active, either because it was dead after initial injection into the channel or because it became exhausted, it does not perform the persistent random walk. Instead, the sperm performs an isotropic random walk (RW). This is equivalent to a PRW where the persistence time P is equal to the time step $\Delta t$. In other words, the sperm moves in a new random direction at every time step by a fixed distance $r_0$, mimicking the Brownian forces from the surrounding media. The diffusion coefficient in this case is given by $D = r_0^2/(4\Delta t)$. This diffusion coefficient can be estimated using the Einstein- Smoluchowski formula [39], i.e. $D = k_B T/\zeta$, where $k_B$ is Boltzmann constant, T is the temperature, and $\zeta$ is the friction coefficient. To determine $\zeta$, the mouse sperm was modeled as a rigid cylinder of length 100 $\mu$m and radius 0.5 $\mu$m, consistent with earlier models and experimental observations. For a cylinder of length L at a distance h from a surface, the friction coefficient along the long axis is given by [41]

$$\zeta \cong \frac{2\pi\eta L}{\ln(2h/r)},$$

where $\eta$ is the viscosity of the medium and r is the radius of the cylinder. To mimic the PBS buffer in the channel, we use $\eta = 10^{-3}$ Pa s, and take h=25 $\mu$m in the above equation, resulting in $\zeta \approx 1.4 \times 10^{-7}$ $kg/s$ at room temperature. Using the Einstein-Smoluchowski formula, this results in a diffusion coefficient of $D \approx 0.03$ $\mu m^2/s$ for the sperm. This means that in a given time step, an inactive sperm will move about 0.3 $\mu$m, before changing its direction.

Since the channel thickness (50 $\mu$m) is much less than the width and length of the channels, the model was restricted to two dimensions. For the walls of the channel, reflective boundary conditions were used, i.e. when a sperm hits a wall, it stops and reflects back at a new random direction.

In the experiments, it was observed that the sperm occupy the first 5 mm of the channel shortly after injection. In order to mimic this effect in the simulations, the sperm were initially distributed randomly with a Fermi-like distribution shown in Fig. 15 and given by

$$N(x) = \frac{N_T}{\mu(e^{\beta(x-\mu)}+1)},$$

where $\mu$ denotes the average location of the interface, and $\beta$ is a parameter that adjusts the sharpness of the initial sperm distribution front, and $N_T$ is the total number of sperm in the channel. It can be shown that $\int N(x)dx = N_T$. In the simulations, the following values were used: $\mu$=5 mm, $\beta$=10 mm$^{-1}$, $N_T$=10$^5$.

**Claims**

1. A method for sorting motile cells, comprising:

   introducing an initial population of motile cells into an inlet port of a microfluidic channel, the initial population of motile cells having a first average motility;
   incubating the population of motile cells in the microfluidic channel in the absence of flowing media; and
   collecting a sorted population of motile cells at an outlet port of the microfluidic channel, the sorted population of motile cells having a second average motility higher than the first average motility; wherein the height of the microfluidic channel is 3 to less than 20 times the dimension of the motile cells, and the length of the microfluidic channel is 12 to 15mm.

2. The method of claim 1, further comprising orienting the microfluidic channel horizontally or vertically; and/or incubating the population of motile cells includes incubating the population of motile cells for a time sufficient to allow a portion of the initial population of motile cells to move along the microfluidic channel.

3. The method according to any one of the preceding claims, further comprising determining the second average motility, including:

   obtaining a plurality of images of a collectable population of motile cells in the vicinity of the outlet port, the collectable population of motile cells including the sorted population of motile cells; and
   analyzing the plurality of images.

4. The method according to any one of the preceding claims,

   wherein introducing the initial population of motile cells includes suspending the initial population of motile cells in a medium at a concentration of at least 10$^3$ motile cells/$\mu$L and
   wherein a concentration of the sorted population of motile cells in a medium is less than or equal to $1.6 \times 10^3$ motile cells/$\mu$L.

5. A method for analyzing a population of motile cells, comprising:

   introducing an initial population of motile cells into an inlet port of a microfluidic channel;
   incubating the population of motile cells in the microfluidic channel in the absence of flowing media;
   acquiring a plurality of images of at least a portion of the population of motile cells within the microfluidic channel; and
   determining a characteristic of at least a portion of the population of motile cells based on the plurality of images;
   wherein the height of the microfluidic channel is 3 to less than 20 times a dimension of the motile cells, and the length of the microfluidic channel is 12 to 15mm.

6. The method of claim 5, wherein the determined characteristic includes at least one of a motility, an average path velocity (VAP), a straight line velocity (VSL), or a linearity;
   wherein the determined characteristic includes at least one of:

   a characteristic of a sorted population of motile cells located in the vicinity of an outlet port of the microfluidic channel, and
   a distribution of the population of motile cells along the length of the microfluidic channel; and/or

   wherein determining a characteristic includes comparing a characteristic of a sorted population of motile cells located in the vicinity of an outlet port of the microfluidic channel with either or both of:

a characteristic of the initial population of motile cells, and
a characteristic of a remaining population of motile cells located in the vicinity of the inlet port after the incubating, and/or further comprising determining a health of the initial population of motile cells based on the determined characteristic.

7. The method according to any one of claims 5 to 6, wherein incubating the population of motile cells includes incubating for a time sufficient to allow a portion of the initial population of motile cells to move along the microfluidic channel

8. The method according to any one of claims 1-2 or 7, wherein the population of cells are incubated for 20-60 minutes, or 30 minutes.

9. The method of claim 1, wherein:

incubating the initial population of motile cells comprises incubating the population of motile cells such that at least some of the motile cells swim from the inlet port to the outlet port of the microfluidic channel;
wherein a third population of motile cells at the inlet port of the microfluidic channel following the incubating has a third average motility and
wherein the second average motility is greater than the third average motility.

**Patentansprüche**

1. Verfahren zum Sortieren von motilen Zellen, das Folgendes beinhaltet:

Einbringen einer Ausgangspopulation von motilen Zellen in eine Einlassöffnung eines mikrofluidischen Kanals, wobei die Ausgangspopulation von motilen Zellen eine erste durchschnittliche Motilität aufweist;
Inkubieren der Population von motilen Zellen im mikrofluidischen Kanal in Abwesenheit fließender Medien; und
Sammeln einer sortierten Population von motilen Zellen an einer Auslassöffnung des mikrofluidischen Kanals, wobei die sortierte Population von motilen Zellen eine zweite durchschnittliche Motilität aufweist, die höher als die erste durchschnittliche Motilität ist; wobei die Höhe des mikrofluidischen Kanals das 3- bis weniger als das 20-fache der Dimension der motilen Zellen beträgt und die Länge des mikrofluidischen Kanals 12 bis 15 mm beträgt.

2. Verfahren nach Anspruch 1, das weiter Folgendes beinhaltet: Ausrichten des mikrofluidischen Kanals horizontal oder vertikal und/oder
Inkubieren der Population von motilen Zellen, einschließlich Inkubieren der Population von motilen Zellen für einen ausreichenden Zeitraum, um es einem Teil der Ausgangspopulation von motilen Zellen zu ermöglichen, sich entlang des mikrofluidischen Kanals fortzubewegen.

3. Verfahren nach einem der vorangehenden Patentansprüche, das weiter das Ermitteln der zweiten durchschnittlichen Motilität beinhaltet, einschließlich:

Gewinnen einer Pluralität von Bildern einer sammelbaren Population von motilen Zellen in der Nähe der Auslassöffnung, wobei die sammelbare Population von motilen Zellen die sortierte Population von motilen Zellen einschließt, sowie
Analysieren der Pluralität von Bildern.

4. Verfahren nach einem der vorangehenden Patentansprüche,

wobei das Einbringen der Ausgangspopulation von motilen Zellen das Suspendieren der Ausgangspopulation von motilen Zellen in einem Medium in einer Konzentration von mindestens $10^3$ motilen Zellen/$\mu$L einschließt; und
wobei eine Konzentration der sortierten Population von motilen Zellen in einem Medium kleiner oder gleich 1,6 x $10^3$ motile Zellen/$\mu$L ist.

5. Verfahren zur Analyse einer Population von motilen Zellen, das Folgendes beinhaltet:

Einbringen einer Ausgangspopulation von motilen Zellen in eine Einlassöffnung eines mikrofluidischen Kanals;
Inkubieren der Population von motilen Zellen im mikrofluidischen Kanal in Abwesenheit fließender Medien;
Erfassen einer Pluralität von Bildern von mindestens einem Teil der Population von motilen Zellen im mikrofluidischen Kanal; und
Ermitteln eines Merkmals von mindestens einem Teil der Population von motilen Zellen basierend auf der Pluralität der Bilder;
wobei die Höhe des mikrofluidischen Kanals das 3- bis weniger als das 20-fache einer Dimension der motilen Zellen beträgt und die Länge des mikrofluidischen Kanals 12 bis 15 mm beträgt.

6. Verfahren nach Anspruch 5, wobei das ermittelte Merkmal mindestens eines aus einer Motilität, einer durchschnittlichen Streckengeschwindigkeit (VAP), einer linearen Geschwindigkeit (VSL) oder einer Linearität einschließt, wobei das ermittelte Merkmal mindestens eines der Folgenden einschließt:

ein Merkmal einer sortierten Population von motilen Zellen, die sich in der Nähe einer Auslassöffnung des mikrofluidischen Kanals befindet, und
eine Verteilung der Population von motilen Zellen entlang des mikrofluidischen Kanals; und/oder
wobei das Ermitteln eines Merkmals den Vergleich eines Merkmals einer sortierten Population von motilen Zellen einschließt, die sich in der Nähe einer Auslassöffnung des mikrofluidischen Kanals befindet, mit einem oder beiden von:

einem Merkmal der Ausgangspopulation von motilen Zellen und
einem Merkmal einer verbleibenden Population von motilen Zellen, die sich in der Nähe der Einlassöffnung nach dem Inkubieren befindet; und/oder weiter beinhaltend das Ermitteln einer Gesundheit der Ausgangspopulation von motilen Zellen auf Basis des ermittelten Merkmals.

7. Verfahren nach einem der Ansprüche 5 bis 6, wobei das Inkubieren der Population von motilen Zellen das Inkubieren für einen ausreichenden Zeitraum einschließt, der es einem Teil der Ausgangspopulation von motilen Zellen ermöglicht, sich entlang des mikrofluidischen Kanals fortzubewegen.

8. Verfahren nach einem der Ansprüche 1 bis 2 oder 7, wobei die Population von Zellen 20 bis 60 Minuten bzw. 30 Minuten lang inkubiert wird.

9. Verfahren nach Anspruch 1, wobei

das Inkubieren der Ausgangspopulation von motilen Zellen das Inkubieren der Population von motilen Zellen in so einer Weise beinhaltet, dass mindestens einige der motilen Zellen von der Einlassöffnung zur Auslassöffnung des mikrofluidischen Kanals schwimmen,
wobei eine dritte Population von motilen Zellen an der Einlassöffnung des mikrofluidischen Kanals nach dem Inkubieren eine dritte durchschnittliche Motilität aufweist und
wobei die zweite durchschnittliche Motilität größer ist als die dritte durchschnittliche Motilität.

**Revendications**

1. Un procédé destiné à trier des cellules motiles, consistant à :

introduire une population initiale de cellules motiles dans un orifice d'entrée d'un canal microfluidique, la population initiale de cellules motiles ayant une première motilité moyenne ;
faire incuber la population de cellules motiles dans le canal microfluidique en l'absence de milieux en écoulement ; et
collecter une population triée de cellules motiles au niveau d'un orifice de sortie du canal microfluidique, la population triée de cellules motiles ayant une deuxième motilité moyenne supérieure à la première motilité moyenne ; la hauteur du canal microfluidique étant de 3 à moins de 20 fois la dimension des cellules motiles, et la longueur du canal microfluidique étant de 12 à 15 mm.

2. Le procédé selon la revendication 1, consistant en outre à orienter le canal microfluidique horizontalement ou verticalement ; et/ou
faire incuber la population de cellules motiles inclut l'incubation de la population de cellules motiles pendant une durée suffisante pour permettre à une partie de la population initiale de cellules motiles de se déplacer le long du canal microfluidique.

3. Le procédé selon l'une quelconque des revendications précédentes, consistant en outre à déterminer la deuxième motilité moyenne, ce qui inclut :

obtenir une pluralité d'images d'une population de cellules motiles pouvant être collectée à proximité de l'orifice de sortie, la population de cellules motiles pouvant être collectée incluant la population triée de cellules motiles ; et analyser la pluralité d'images.

4. Le procédé selon l'une quelconque des revendications précédentes,

dans lequel l'introduction de la population initiale de cellules motiles consiste à suspendre la population initiale de cellules motiles dans un milieu dans une concentration d'au moins $10^3$ cellules motiles/$\mu$L et dans lequel une concentration de la population triée de cellules motiles dans un milieu est égale ou inférieure à 1,6 x $10^3$ cellules motiles/$\mu$L.

5. Un procédé destiné à trier analyser une population de cellules motiles, consistant à :

introduire une population initiale de cellules motiles dans un orifice d'entrée d'un canal microfluidique ; faire incuber la population de cellules motiles dans le canal microfluidique en l'absence de milieux en écoulement ; acquérir une pluralité d'images d'au moins une partie de la population de cellules motiles dans le canal microfluidique ; et déterminer une caractéristique d'au moins une partie de la population de cellules motiles sur la base de la pluralité d'images ; la hauteur du canal microfluidique étant de 3 à moins de 20 fois une dimension des cellules motiles, et la longueur du canal microfluidique étant de 12 à 15 mm.

6. Le procédé selon la revendication 5, dans lequel la caractéristique déterminée inclut au moins une caractéristique parmi une motilité, une vitesse de parcours moyenne (VAP), une vitesse de ligne droite (VSL) ou une linéarité ; dans lequel la caractéristique déterminée inclut :

soit une caractéristique d'une population triée de cellules motiles située à proximité d'un orifice de sortie du canal microfluidique, soit une distribution de la population de cellules motiles le long de la longueur du canal microfluidique ; et/soit dans lequel la détermination d'une caractéristique consiste à comparer une caractéristique d'une population triée de cellules motiles située à proximité d'un orifice de sortie du canal microfluidique avec :

soit une caractéristique de la population initiale de cellules motiles, et/soit une caractéristique d'une population restante de cellules motiles située à proximité de l'orifice d'entrée après l'incubation, et/ou consistant en outre à déterminer la santé de la population initiale de cellules motiles sur la base de la caractéristique déterminée.

7. Le procédé selon l'une quelconque des revendications 5 à 6, dans lequel l'incubation de la population de cellules motiles inclut l'incubation pendant une durée suffisante pour permettre à une partie de la population initiale de cellules motiles de se déplacer le long du canal microfluidique.

8. Le procédé selon l'une quelconque des revendications 1-2 ou 7, dans lequel la population de cellules est incubée pendant 20 à 60 minutes, ou 30 minutes.

9. Le procédé selon la revendication 1, dans lequel :

l'incubation de la population initiale de cellules motiles consiste à faire incuber la population de cellules motiles de telle sorte qu'au moins certaines des cellules motiles nagent de l'orifice d'entrée à l'orifice de sortie du canal microfluidique ; dans lequel une troisième population de cellules motiles au niveau de l'orifice d'entrée du canal microfluidique à la suite de l'incubation a une troisième motilité moyenne et

dans lequel la deuxième motilité moyenne est supérieure à la troisième motilité moyenne.

Fig. 1

Fig. 2A

Fig. 2B

EP 2 710 139 B2

Fig. 3

Fig. 4

Fig. 5A

Fig. 5B

Fig. 6A

Fig. 6B

Fig. 6C

Fig. 7

Fig. 8

Figs. 9A-D

Fig. 10

Figs. 11A-D

Fig. 12

Fig. 13

EP 2 710 139 B2

F

Fig. 14

Fig. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61488300
- US 2010291535 A1
- US 2008187991 A1
- US 2006270021 A1
- US 20050026274 A

**Non-patent literature cited in the description**

- **ZHANG et al.** Lab on a Chip. The Royal Society of Chemistry, 2011, vol. 11, 2535-2540
- **XIE et al.** *Clinical Chemistry,* 2010, vol. 56, 8
- **OZCAN ; DEMIRCI.** *Lab Chip,* 2008, vol. 8, 98-106
- **MEIJERING, DZYUBACHYK.** Smal. Methods for Cell and Particle Tracking. Methods in Enzymology. February 2012, vol. 504, 183-200